# EUROPEAN PATENT APPLICATION

(11) **EP 4 124 306 A1**
(43) Date of publication of application: **01.02.2023**
(21) Application number: 22187706.1
(22) Date of filing: 29.07.2022
(51) Int. Cl.: A61B 17/221, A61B 17/22

(54) **DUAL LAYER ICAD DEVICE**

(30) Priority: 30.07.2021 US 202117444061
(71) Applicant: Neuravi Limited, Galway H91 K5YD (IE)
(72) Inventor: CASEY, Brendan, Galway, H91 K5YD (IE); GILVARRY, Michael, Galway, H91 K5YD (IE)
(74) Representative: Carpmaels & Ransford LLP

(57) **Abstract**

A design bringing together mechanical thrombectomy and stenting for more efficient treatment of occlusions and stenotic lesions in the cerebral vasculature features a dual-layer setup. The outer layer consists of an expandable stent cage with large cell openings which can allow thrombus to pass through the openings into the inner expandable capture section layer. The clot capture section can be configured to grip and extract the occlusion. The outer cage is configured to be detached from the remainder of the device and left implanted as a stent to exert an outward radial force to support and/or dilate the region of stenosis. The device can have a distal fragment protection element to prevent the distal migration of elements liberated during the procedure.

## Description

### Cross-Reference to Related Applications

This application is a Continuation-In-Part of U.S. Application No. 16/510,652, filed July 12, 2019, which is a Continuation of U.S. Application No. 14/206,973, now Patent No. 10,390,850, filed March 12, 2014, which claims priority to United States Provisional Patent Application No. 61/785,213 filed March 14, 2013. This application is also a Continuation of U.S. Application No. 16/774,191, filed January 28, 2020. The contents of these applications are incorporated herein by reference in their entirety as if set forth verbatim.

### Field of the Invention

The present invention generally relates devices and methods used in removing obstructions and treating stenosis in the cerebral blood vessels during intravascular medical treatments. More specifically, the present invention relates to a device which can be used as a standard clot retrieval device while also having a detachable element which can be left implanted in the stenotic lesion if required.

### Background

Atherosclerosis results from lesions which narrow and reduce the space in the lumen of vessels in the vasculature. Such lesions are usually composed of plaque, which can be fat, cholesterol, calcium, or other components of the blood. Severe occlusion or closure can impede the flow of oxygenated blood to different organs and parts of the body and result in other cardiovascular disorders such as heart attack or stroke. Narrowing of vessels, or stenosis, can cause clots to form in-situ and also increases the risk that clots and other emboli can lodge at such locations, especially in the neurovascular where vessel diameters are already small. Intracranial atherosclerotic disease (ICAD) is the narrowing of those arteries and vessels supplying blood to the brain and represents a common proximate mechanism of ischemic stroke.

Treatment for vascular occlusions are well known in the art. Methods can include utilizing drugs, such as anticoagulants or anti-platelet agents, as well as medical procedures such as surgical endarterectomy, angioplasty, and stenting. Much of the recent success in endovascular revascularization treatments (ERT) has been the further development of safe thrombectomy devices. Devices such as stentrievers, direct-aspiration systems, and other clot retrieval devices have been strongly associated with better clinical outcomes. However, these devices are primarily designed to recanalize the vessel by removing and retrieving an occluding embolus. Sufficient recanalization may not occur if there is also significant stenosis present at the occlusion site, increasing the need for implanted stents.

Treatment methods for the neurovascular in particular depend on multiple factors, including the degree of occlusion, the shape of the target occlusion site (i. e. truncal, branching, etc.), and the patient's overall condition. For these reasons it can be unknown if the occlusion is the result of a blood clot alone of if a stenosis is also present. During the treatment of stroke or transient ischemic attack, identifying stenotic lesions can be arduous because it is difficult to differentiate them from clots and other embolism-related occlusions through baseline angiography. This is especially in the very small and tortuous vessels of the cerebral vasculature. In some instances, a physician may not be able to identify a stenotic lesion until the operation is underway. The physician may therefore need to have various readily accessible treatment devices available to be able to dynamically adapt based on the diagnosis, including stenting systems.

Procedure times are compounded by these difficulties, and in cases where both a blood clot and stenosis are present, the physician is often required to change out catheters, devices, and guidewires after removing the clot. The need for multiple passes and device deliveries during treatment can increase the likelihood that the ICAD lesion ruptures or fragments released. Such fragments can include but are not limited to blood clots, plaque, and other thrombi debris. The fragments can lead to vascular occlusions causing extensive stroke or death.

The need for shorter door-to-procedure times is always present to limit lasting damage in ischemic stroke patients. Due to the difficulty in diagnosing ICAD, a physician may need to cross the lesion multiple times during a treatment, thereby further increasing the likelihood of rupture or fragmenting and lengthening the procedure. There therefore remains a need for systems and devices with improved performance to address intravascular occlusions, particularly ICAD lesions in the neurovascular.

### Summary

It is an object of the present design to provide systems, devices, and methods to meet the above-stated needs. Having the ability to conduct mechanical thrombectomy and stenting operations with a single device or system, such as the current design, can greatly reduce procedure times and thus result in better clinical outcomes. This is particularly true in the case of stroke patients, where the degree of recovery is greatly dependent on timing. Furthermore, for cumbersome cases where the occlusion is thrombotic or atherosclerotic and clot retrieval is unsuccessful, the current design greatly improves procedural flexibility by allowing the stent to be immediately deployed after a capture attempt to maintain a flow lumen through the clot and scaffold the lesion.

Generally, the proposed system provides for a dual-capability setup for addressing an occlusion or obstruction in a blood vessel. The occlusion can be one or more of a clot, thrombus, lesion, or other embolisms. The system can be deployed across an occlusion as per a standard stent or thrombectomy device in a single microcatheter. The outer layer consists of a stent cage with an open distal mouth and large cell openings which can allow thrombus to pass through the openings into the inner capture section layer. The cage can also expand the walls of the lesion with its radial force to serve as a temporary stent. The device can be retracted into an access catheter to retrieve the thrombus under aspiration from the aspiration or access catheter. Multiple retrieval passes can be conducted until satisfactory recanalization has occurred. Once the obstruction has been cleared, the outer stent cage can either be removed from the patient or redeployed to the target and detached as a permanent stent implant.

An example device for removing an obstructive occlusion from and stenting a blood vessel of a patient can include an inner capture section and an outer stent cage which are deliverable from a microcatheter. The microcatheter can be navigated to a target site in the neurovascular using standard interventional techniques and commercially available ancillary devices such as an access catheter, balloon guide catheter, and/or guidewires. The capture section of the device design can be used to retrieve an obstructive clot or thrombus and can have an elongate body with a proximal end, a distal end, a collapsed delivery configuration, and an expanded deployed configuration. The capture section can include a strut framework or truss which forms an outer network of closed cells. When the capture section is deployed from the collapsed delivery configuration, at least a portion of this strut framework can be configured engage the clot in the expanded state. In another case, the device can transition from the expanded deployed configuration to a partially-constrained pinching configuration, where one or more portions of the occlusion can be pinched between peaks of the strut framework of the capture section when the device is drawn back into an outer catheter. Applied aspiration during the procedure can assist in dislodging the clot from the vessel.

The stent cage of the device can also have a collapsed delivery configuration and a self-expanded deployed configuration and can be expandable to a greater radial extent than the capture section when in the self-expanded deployed configuration. The cage can include a plurality of interconnected struts formed in to a network of large circumferential cells. The cells are large enough so there is little resistance to the thrombus, clot, or other emboli migrating into a reception space in the interior of the outer cage. The stent cage could also possess one or more sections extending between its proximal and distal ends where two or more struts of the plurality of struts are longitudinally aligned. In one example, a maximum radial dimension of the stent cage when expanded can be approximately equal to a maximum radial dimension of the expanded capture section. In one configuration the struts of the stent cage and capture section longitudinally align so that a thrombus can easily pass through the cage and capture section. In some instances, the stent cage can be two or more times the diameter of the capture section. When in the expanded deployed configuration, the stent cage can also be sized to have a maximum radial dimension larger that a maximum radial dimension of the target blood vessel such that the stent cage exerts an outward radial force on the vessel when deployed to increase the localized size of the lumen. Such a configuration allows the outer cage to provide dilation and support for the vessel as the capture section and any liberated material is retrieved and withdrawn from the patient.

The stent cage can be a substantially tubular shape and configured to be at least partially eccentric to the capture section. In one configuration, the stent cage can have an open distal mouth with flared circumferential edges. The flared edges can help to restrain and hold a clot into which the device has been deployed. At its proximal end the stent cage can have a collar or detachment point where the cage can be released from the rest of the device and left implanted as a stent when the capture section is removed. The capture section and stent cage of the device can be delivered on a single actuatable shaft. In an alternate example, the cage and capture section can be longitudinally moveable independent of one another, and each can have their own proximal shaft for manipulating the cage and capture section within the patient.

Mechanical thrombectomy procedures always carry the risk that fragments liberated during the procedure can migrate distally and become additional emboli. Often aspiration from one or more catheters is used to reverse flow direction, but other protective measures can also be employed. In one aspect of the current design a fragment protection element can be attached to the device tethered to and distal of the capture portion to serve as a protective structure across the vessel lumen to occupy space and substantially block the distal egress of fragments. The element can be three-dimensional such that it has depth and a surface area larger than the cross section of the capture portion of the device. The protection element can have a proximal connecting member allowing it to float in the vessel distal of the remainder of the device or float distal of the capture portion inside the outer cage. The protection element can have a collapsed delivery condition and can deploy to expand to a radial extent greater than that of the expanded capture section. A fragment filter at the distal end can take on a number of porous forms, such as a mesh filter, a basket, a series of fibers or fine wires, or a series of struts arranged in a volumetric pattern.

In another example, a thrombectomy device capable of removing obstructions and treating stenotic lesions can have an outer cage and a retrieval device. The outer cage can be a substantially tubular structure having a maximum radial size approximately equal to or larger than a maximum radial size of the retrieval device. The outer circumference of the cage can have a lattice of interconnected struts forming a network of closed cells. The network of cells can be of sufficiently low density such that the thrombus or fragments are displaced and encouraged into an open interior reception space of the cage. The outer cage can have a constrained delivery configuration when folded inside a microcatheter. The cage can also be constructed of a shape memory allow such that it has a preset expanded deployed configuration. When deployed the outer cage can be configured to exert an outward radial force on a vessel with a lumen having a maximum radial size less than the maximum radial size of the outer cage. In one example, the maximum radial size of the outer cage can be approximately 6.0 mm in diameter.

The maximum radial size of the outer cage could also flare to a greater radial diameter at the distal end to provide the largest opening to improve retrieval capabilities. The outer cage can also be configured to be detachable from the rest of the device to remain implanted in a stenotic lesion. The implant would scaffold the lesion and maintain a flow lumen through the vessel.

The retrieval device can be at least partially disposed within the lumen of the outer cage. The retrieval device can have a longitudinally-extending elongate body having a proximal end, a distal end, and a multitude of struts which can converge at terminal connections to form adjacent segments along the length of the body. The retrieval device can have a constrained delivery configuration and an expanded deployed configuration. The struts of the adjacent segments can be configured to engage at least a portion of the vessel obstruction in the expanded deployed configuration. and can embed, constrain, or pinch to gain a strong grip on the clot or thrombus for the initial step of disengaging it from the vessel.

The treatment device can have a first proximal shaft for articulation of the retrieval device and a second proximal shaft capable of articulating the outer cage. Being on separate shafts allows movement of retrieval device and outer cage independent of the other and enables the retrieval device to be withdrawn on its own once a firm grip on the thrombus is obtained. Alternatively, a single shaft could be used where the retrieval device and outer cage are deployed and withdrawn together as a unit. Upon capturing a clot, the retrieval device can either be withdrawn fully through the lumen of an outer catheter or can be drawn back far enough to lodge and pinch a firmer thrombus in the tip of the outer catheter or microcatheter to be withdrawn in tandem.

When the outer cage is detached and released the proximal struts of the outer cage can expand from the point of detachment so that the cage assumes a generally tubular shape to engage and appose the lesion and vessel wall and the cage can remain in the vessel as an implanted stent.

In another case, the device can further have a distal fragment protection element fixedly connected to the distal and of the retrieval device by a shaft or other connecting element. The protection element can protect against embolization and can be a distal net or scaffolding zone across the space of the lumen. Fibers or fine wires can be added to the scaffolding without impacting the profile or deliverability of the device. Fragments caught by the protection element are withdrawn from the patient together with the retrieval device.

Also provided is a method bringing together mechanical thrombectomy and stenting procedures. The method can have some or all of the following steps, and the steps are not necessarily in the recited order. Access to a patient's vasculature can be obtained using conventionally known techniques. An access catheter and a microcatheter with hollow internal lumens are directed to a target site in the cerebral vascular where an obstructive thrombus is lodged in a region of stenosis. Delivered through the microcatheter is a self-expanding outer cage sized to exert a radial force on the walls of the target vessel when in an expanded, deployed state. The outer cage can have a plurality of large cells around the circumference and a detachment point at its most proximal end. An expandable capture portion is disposed with the outer cage which has a maximum radial size when expanded less than a maximum radial size of the outer cage. In an alternative example, the capture portion could have an expanded maximum radial size that was the same or similar to the maximum radial size of the outer cage. In another alternative, the capture portion could have an expanded maximum radial size that was slightly larger than the maximum radial size of the outer cage, such that the outer cage would constrain the capture section when fully expanded. A fragment protection element can also be fixedly connected or via a shaft or wire to the distal end of the capture portion.

Aspiration can be applied through one or more catheters used in the procedure. The microcatheter can be advanced across the thrombus using a guidewire and standard techniques, the device introduced, and the outer cage and capture portion deployed to expand in the vessel. Radiopaque markers, coatings, or other options known in the art can be used along the length of the device, or at the proximal and distal ends of the outer cage, to mark the terminal points of device during the procedure. The cells of the outer cage circumference are porous for displacing and receiving the thrombus and lesion fragments to the interior of the cage. The capture section can be expanded to grip and capture the thrombus. The outer cage and capture section with the captured thrombus can then be withdrawn back in to the access catheter with aspiration.

The outer cage and capture section can be advanced repeatedly across the thrombus for additional capture attempts as necessary to clear the vessel. When the thrombus has been cleared, the outer cage and capture section can be re-deployed to the region of stenosis at the target site. The outer cage can then be detached to remain in the vessel as an implanted stent to protect the flow lumen and dilate the vessel.

Alternately, the outer cage can be released and implanted first, followed by the capture section and captured thrombus being withdrawn back in to the access catheter and removed from the patient.

The method could also incorporate the step of positioning a distal fragment protection element approximate the distal end of the device. The fragment protection element can be fixedly connected to the distal and of the retrieval device by a tethered shaft or other connecting element. The protection element can remain in place during the procedure and the deployment of the stent cage as an implant to protect against embolization until removed with the capture section.

Other aspects and features of the present disclosure will become apparent to those of ordinary skill in the art, upon reviewing the following detailed description in conjunction with the accompanying figures.

### Brief Description of the Drawings

The above and further aspects of this invention are further discussed with the following description of the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation. It is expected that those of skill in the art can conceive of and combining elements from multiple figures to better suit the needs of the user.
Fig. 1 is a dual-purpose thrombectomy and stenting device according to aspects of the present invention;
Fig. 2 is a detailed view of the device of Fig. 1 according to aspects of the present invention;
Fig. 3 shows the outer cage of the device from Fig. 1 according to aspects of the present invention;
Fig. 4 illustrates the outer cage of the device from Fig. 1 as implanted in a target vessel according to aspects of the present invention;
Fig. 5 shows the capture section and fragment protection element of the device from Fig. 1 according to aspects of the present invention;
Fig. 6 shows a front profile view Fig. 5 according to aspects of the present invention;
Fig. 7 shows another dual-purpose thrombectomy and stenting device according to aspects of the present invention;
Fig. 8 shows the device from Fig. 7 deployed in a vessel with an occlusive thrombus and a region of stenosis according to aspects of the present invention;
Fig. 9 shows another dual-purpose thrombectomy and stenting device according to aspects of the present invention;
Fig. 10 shows a further dual-purpose thrombectomy and stenting device according to aspects of the present invention;
Figs. 11a - 11e show alternative configurations for the fragment protection element according to aspects of the present invention;
Figs. 12a - 12d show flow steps for the use of a device for conducting a combined mechanical thrombectomy and stenting procedure according to aspects of the present invention;
Figs. 13 and 14 are flow diagrams outlining a method for using the device to conduct mechanical thrombectomy and stenting operations according to aspects of the present invention.

### Detailed Description

Specific examples of the present invention are now described in detail with reference to the Figures, where identical reference numbers indicate elements which are functionally similar or identical. It is an object of the current invention to offer a system or device which gives the physician the advantage of operational flexibility to adapt to complications or unknowns in an intravascular procedure, such as when an occluded vessel has a blood clot and also a region of underlying stenosis which was not detected during angiography. These improvements can lead to safe and more rapid access to complex areas of the intercranial arteries to remove occlusions and shorten procedure times.

Accessing the various vessels within the vascular, whether they are coronary, pulmonary, or cerebral, involves well-known procedural steps and the use of a number of conventional, commercially-available accessory products. These products, such as angiographic materials, rotating hemostasis valves, and guidewires are widely used in laboratory and medical procedures. When these products are employed in conjunction with the system and methods of this invention in the description below, their function and exact constitution are not described in detail. While the description is in many cases in the context of treating intercranial arteries, the systems and devices may be used in other body passageways as well.

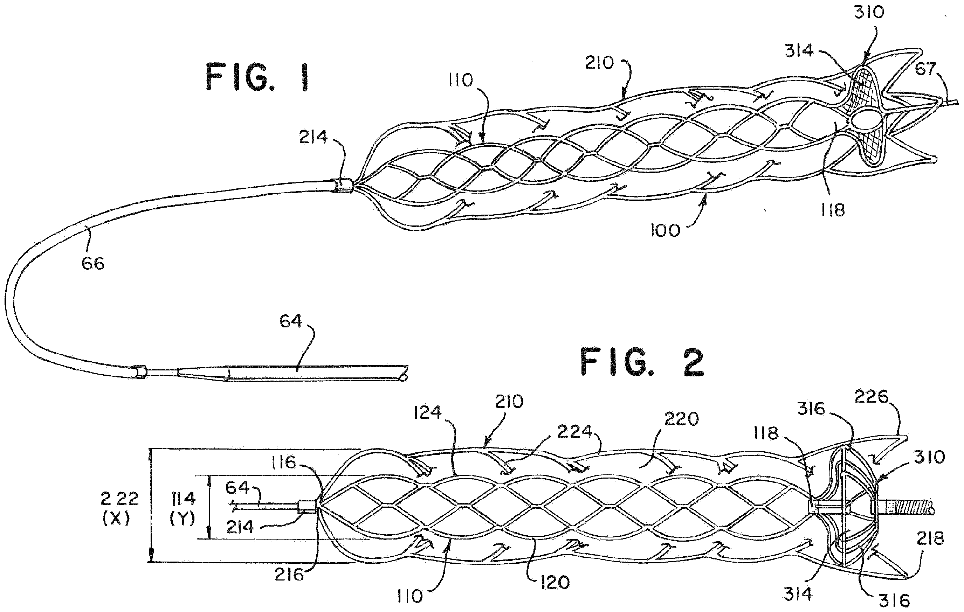

Turning to the figures, Fig. 1 illustrates a device 100 which can include an inner capture section 110 having a collapsed delivery configuration and an expanded deployed configuration and an outer stent cage 210 at least partially overlapping with the inner capture section 110 longitudinally. The outer stent cage 210 is also radially expandable and may assume a maximum radial size that is less than, similar to, or greater than a maximum radial size of the capture section 110 when deployed. The device can have a proximal elongate shaft 64 to allow manipulation by the user. The device can be delivered to a target site for a mechanical thrombectomy procedure using standard techniques with a guide sheath, access catheter, microcatheter, or other delivery system (not shown). The device can further include a fragment protection element 310 which can be deployed distal to the capture section which provides a physical barrier to possible debris liberated during a procedure.

A detachment point 214 can be provided for the device 100 approximate the proximal end of the stent cage 210. The detachment point can be remotely actuated by the user to detach the stent cage from the remainder of the device. Any number of methods can be deployed for detachment such that the stent cage can be physically decoupled from the remainder of the system or device.

At least a part or parts of the capture section 110 and stent cage 210 can be made from Nitinol or another shape memory material with sufficient elastic strain capacity such that the elastic limit would not be exceeded when the device 100 was in the collapsed delivery configuration within a delivery system. This strain capacity allows the device to be effectively "spring loaded" within the microcatheter or delivery catheter so that it can self-expand to engage a clot when deployed out of the distal end of the delivery system.

Parts of the device 100 could be rendered visible under fluoroscopy by the addition of alloying elements or with the inclusion of radiopaque markers or coatings. For example, the device can have a distal radiopaque coil 67 and a proximate radiopaque coil 66 approximate the terminal ends of the capture section during a thrombectomy procedure.

Fig. 2 is a side view of the device of Fig. 1. At least some portion of the clot capture section 110 and filter element 314 can reside in the lumen of the stent cage 210. The capture section can be a stentriever and the longitudinal axis 228 of the capture section 110 can be coincident with or offset from the longitudinal axis 228 of the stent cage 210. The distal end 218 of the stent cage can be an open mouth, or it can taper down in a more conical profile. If open, there is no distal connection point between the stent cage 210 and the capture section 110. This can allow the capture section to move in a radial direction when it interfaces with a clot

The clot capture section 110, stent cage 210, and a filter element 314 of the fragment protection element 310 can have a collapsed configuration for delivery and an expanded configuration for clot retrieval, flow restoration, and fragment protection. The elongate shaft 64 of the device can be a tapered wire shaft, and be constructed of stainless steel, MP35N, Nitinol, or other material with sufficiently high tensile strength and modulus to allow responsive and consistent trackability of the device in the vascular.

The distal portions of the stent cage 210, clot capture section 110, and fragment filter element 314 together define a three-dimensional protective structure to substantially prevent the distal egress of a clot or clot fragments from the device. The fragment filter element 314 can take on any of a number of forms and can have a structure with a surface which can be configured as a clot barrier surface. In one example, the fragment filter can have spoked arrangement made up of a combination of struts 316. In other cases, a strut framework can be covered with a fibrous mesh or weave that could be permeable to liquid in the vessel but prohibit the distal migration of larger solids. This protective structure prevents the egress of a clot or clot fragments that have entered the inner capture section 110 or reception space 220 between the inner clot capture section and the outer stent cage frame 210.

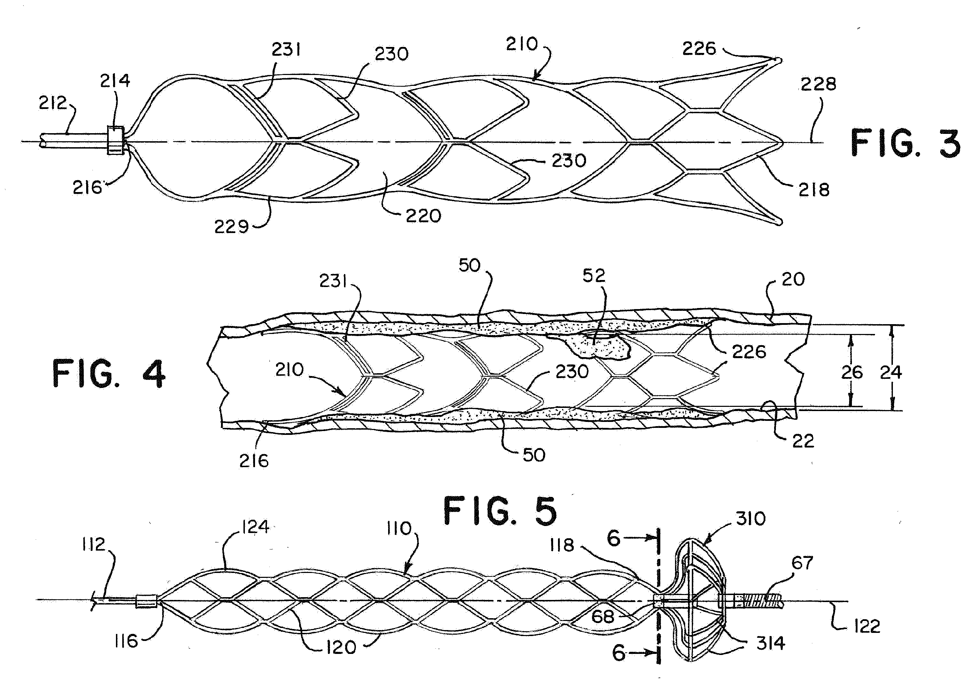

The outer expandable stent cage 210 of the example from Fig. 1 is illustrated in Fig. 3. The cage can have a distal end 218, a proximal end 216, and a proximal shaft 212 allowing manipulation. In one example, the stent cage 210 can have a series of interconnected struts 229, 230, 231 which can define a substantially tubular structure about a longitudinal axis 228. Some struts 230 can terminate in crowns with no distal connecting elements while other struts 231 can terminate injunction points. The stent cage 210 can also have connecting arm struts 229 which define an outer surface largely parallel to the longitudinal axis 228. The struts can form an outer circumferential network of closed cells through which a clot or clots can be displaced radially inward to enter a reception space 220.

The distal end 218 of the stent cage 210 can be defined by a series of crowns or distal undulating struts 226. The undulating struts can flare with a large bend radius for atraumatic contact with vessel walls. The struts at the proximal end 216 of the stent cage can taper down to a detachment point 214 on the proximal shaft 212. The detachment point can be configured to allow the stent cage to be detached from the rest of the device to remain implanted in the patient as a stent, such as in an ICAD lesion.

The detachment point 214 of the stent cage 210 can have one or more collars, partial collars, or sleeves which could be assembled over step features in the shaft 212 such that they form a mechanical lock which could prevent joint disassembly during tension or compression. Alternatively, the collars could be assembled over a notch on the capture portion 110. To detach the stent cage, the detachment point could be actuated through mechanical, electrical, or other means, allowing the stent cage to scaffold a region of stenosis. Once released from the detachment point the stent cage is held in place in the vessel through the outward radial force imparted on the vessel walls.

In another example, the stent cage can be a stent having a plurality of resilient metal strands formed in a braided or mesh pattern. The strands or struts of the stent can extend longitudinally and be woven in a largely helical configuration with the central axis or centerline of the resulting tubular structure as a common axis. A first set of strands can be wound in one direction while being axially displaced from one another. A second group of strands could be wound in the opposite direction from the first while also being axially displaced relative to each other.

When elements described and visualized in the figures as a tubular structure and generally illustrated as a substantially right cylindrical structure, when used herein, the terms "tubular" and "tube" are to be construed broadly. They are not meant to be limited to a structure that is a right cylinder or strictly circumferential in cross-section or of a uniform cross-section throughout its length.

The stent cage 210 can be constructed so that when deployed it expands to a predetermined outer diameter and has sufficient radial force to provide good embedding in the lesion while displacing a thrombus into the reception space. Low levels of scaffolding in the stent cage can be achieved by minimizing the potential surface contact area between the interconnected cage struts 224 and the thrombus or lesion. In one example, a less dense cell network with fewer struts can be utilized. In another example, struts of the cage structure can curve radially inward towards the longitudinal centerline or axis of the device in an oscillating periodic fashion to gain a localized reduction in force and contact area with the thrombus. Localized reductions could also be gained by having multiple aligned axially and/or radially between the distal end 218 and the proximal end 216 of the cage.

The raw material for the stent cage 210 could take many forms such as wire, strip, sheet, or tube. The expandable body of the stent cage can be made from a material capable of recovering its shape automatically once released from a highly strained delivery configuration. A superelastic shape memory material such as Nitinol or an alloy with similar properties is particularly suitable. These materials have sufficient elastic strain capacity such that the elastic limit would not be exceeded when the cage is constrained in the collapsed delivery configuration within a microcatheter or outer access catheter. This elastic strain capacity allows the cage to be effectively spring-loaded within the outer catheter so that it can self-expand when deployed out of the distal end of the outer catheter. In a separate case, the framework could be constructed from wire, allowing a non-superelastic material like a stainless-steel alloy to be employed, since the wires would be free to move independent of one another.

In one example, a Nitinol tube or sheet could be laser-cut and then heat set to form a framework of struts and connecting members. This tubular structure can be heat treated on a mandrel to a suitable temperature to impart stress-relief on the structure, cause the tube to conform to the shape of the mandrel. In these ways the elastic properties of the stent braid can be controlled such that the stent can aid in the implantation process and maintain stiffness and strength over the desired lifetime of the implant. The winding of the braid strands can also be sufficiently dense to provide a stable configuration capable of supporting the full inner diameter of a vessel when implanted.

The stent cage 210 can be bare metal, or the material can be coated with a non-pharmacological coating such as silicon carbide, carbon, and titanium-nitride-oxide. The coating can be hydrophilic or have additives effective to increase the lubricity of the mesh braid of the stenting device 112 to allow for more atraumatic navigation of the vasculature. In another example, the coating could be hydrogel or include soluble particles in a polymeric matrix which could soften or fully dissolve when exposed to an aqueous medium like blood. In other cases, stents have been coated with biodegradable, drug eluting coatings designed to inhibit restenosis. For example, these could be anti-platelet or anti-coagulative agents. These agents could elute from the matrix of the coating when exposed to aqueous media and help prevent the implanted stent 128 from forming a potential nidus for future clot formation.

The interconnected struts 224 can be formed as a monolithic structure or can be assembled as a combination of substructures. The distalmost crowns or undulating struts 226 can have a flared profile. The reception spaces 220 in the lumen of the stent cage can be made sufficiently large such that there is amply room when a thrombus is urged through or by the circumference of cells without significantly compressing it and altering the coefficient of friction.

In the majority of cases, the device can be used strictly to conduct thrombectomy procedures. However, in perhaps 40% of cases involving certain patient populations, it may be desirable to implant a stent to scaffold and dilate a located region of stenosis. Following recanalization, the detachment point can be configured to release the stent cage from the remainder of the device. The outward radial force provided by the stent cage on the walls a vessel could allow it to remain static could be used to provide localized support or even dilation for the vessel in order to maintain the fidelity of the lumen flow path. Once the stent cage has been implanted as a stent the remainder of the device, including the capture section 110 and fragment protection element 310, could be removed from the patient.

Fig. 4 shows an instance where the stent cage 210 is a stent implanted in a blood vessel 20 to dilate a region of stenosis 50. The radial size of the stent cage can be selected to tailor the exerted radial force such that the radial size 26 of the vessel lumen 22 in the lesion is restored to some percentage of the nominal radial dimension 24 of an unaffected area adjacent to the lesion. The percentage can depend on the amount of plaque accumulated in the lesion. Friction from the radial force exerted by the expanded stent cage can maintain the longitudinal location of the stent within the vessel.

The capture section 110 can have a proximal elongate body 124 and a distal filter element 314 for fragment protection, as illustrated in Fig. 5. The elongate body can a proximal end 116, a distal end 118, a longitudinal axis 122, and a proximal shaft 112. The longitudinal axis 122 may or may not be coincident with the longitudinal axis 228 of the stent cage when the device is deployed. The elongate body can take on a number of shapes and may have tapered sections, flared sections, and open or closed ends. The elongate body can be configured to have an expanded diameter in the region of approximately 1.5mm to 5.0mm. The elongate body can have an expandable framework of struts 120 or crowns configured to grip and remove an occlusive clot. The struts can have bends which form necked regions enabling differing longitudinal sections of the elongate body to exert varying radial forces on the clot. A variable radial force allows the elongate body to establish a firmer grip on the clot between sections, making it easier to initially disengage from the vessel walls. The filter element can radially expand to a greater extent than the elongate body 124 in the deployed condition.

In another example, when expanded to the deployed configuration the elongate body 124 of the capture section 110 can have a substantially tubular shape disposed around a longitudinal axis 112 and can be configured to exert a strong radial force to open a flow lumen through a clot. This small flow path could restrict the initial flow so that the supply of blood is gradually reestablished to the affected area to reduce the risk of reperfusion injury. Alternately, the elongate body 124 of the capture section 110 can have a planar structure which can oscillate periodically or extend in a spiral around the longitudinal axis 112. Configured this way, the elongate body could expand and contract in response to the forces of the thrombectomy procedure and grip or pinch a clot between adjacent peaks of the structure for extraction from the vessel.

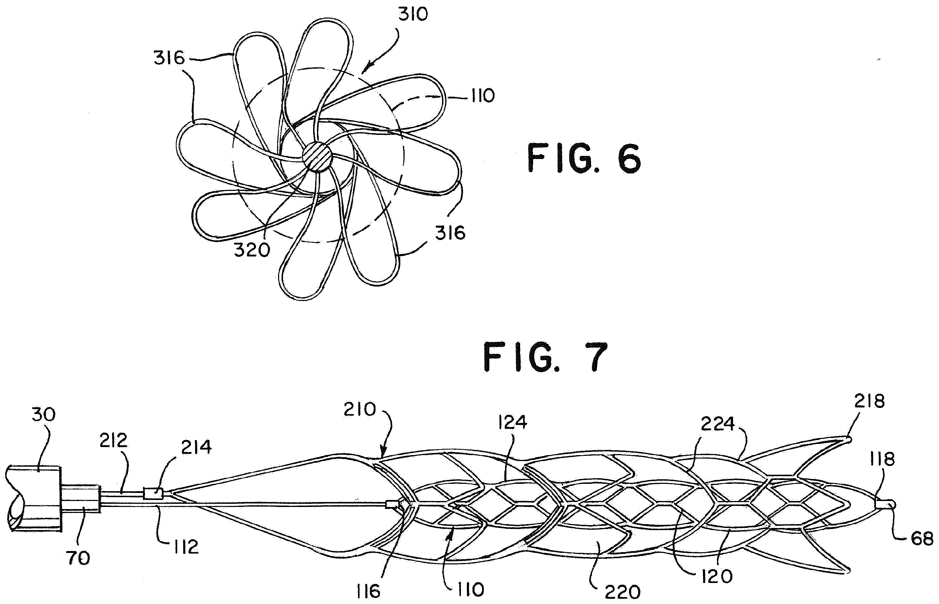

Fig. 6 is an end-on view of the capture section 110 and fragment protection element 310 of the example in Fig. 5 in the expanded deployed configuration. In one configuration, at least a portion of the fragment protection element can expand to a greater degree than the clot capture section so as to provide a downstream surface to intercept liberated clot fragments. In the example shown, the fragment protection element can have a framework of struts 316 extending radially outward in a spoke-type arrangement from a central fragment protector hub 320 to form a physical barrier for preventing the distal migration of debris from the thrombectomy procedure. The strut framework can be of flexible or rigid construction or the struts could be configured to hinge or fold about a plane to occupy more space downstream of the capture section. The hub can be a simple tubular member extending distally from the capture section or could be a wound wire bundle or mesh. The struts 316 of the framework can have flared or bulged sections and can be configured in an overlapping spiral pattern for increased fragment protection.

Like some previous examples, further designs for thrombectomy and stenting having dual expandable members are disclosed. In Fig. 7 a device is illustrated which can have a first inner expandable member which can serve as a clot capture section 110 and a second outer expandable member 210 which can serve as a stent. The inner member can be a stentriever arranged substantially within the lumen of the outer member. The inner member can have an expandable framework of struts or crowns configured to grip and remove an occlusive clot. The properties of the inner and outer members can be tailored independently of one another. For example, in designs where both members comprise connected struts, the shape and level of porosity of the inner member strut framework 120 can be very different than that of the outer member interconnected struts 224. In this way the stiffness and radial force of the inner member can be configured to grip a clot while the outer member is configured to scaffold the vessel.

The receiving openings of the outer cage 210 can be large such that they offer little resistance to motion of the clot relative to the cage. The clot can be locally compressed as the outer stent cage is expanded and the receiving openings allow the thrombus or debris to escape the compression by displacing into the interior reception space 220 of the cage. This reduces the radial force imparted on the vessel by the stent cage in the region of the stenosis which means a lesser force is required to retrieve the thrombus, which in turn results in less vessel trauma and tension on the distal vascular bed. The radial force of the cage can act strongly at small diameters, similar to a compressed spring, to displace the thrombus. The radial force can be weaker at larger diameters to gently press the lesion and vessel walls.

The distal end 118 of the inner member capture section 110 can extend distally of the distal end 218 of outer cage 210. Applied aspiration can urge a clot proximally to be engaged by the strut framework 120 of the capture section.

The inner and outer members can be delivered from the same microcatheter 70 and directed to a target site in the patient's vascular through the lumen of a guide or access catheter 30. The device can have separate shafts allowing for independent movement of the inner and outer members. A first proximal shaft 112 can be capable of articulating the capture section 110 and a second proximal shaft 212 can be capable of articulating the stent cage 210. Having independent shafts can allow the user to adapt to situations where, for example, the axial location for grasping the clot is slightly different from the axial position that is most effective for restoring a flow path. The use of separate shafts can allow the clot to be retrieved independently of the deployment of the stent cage. After clot retrieval, the physician could elect to leave the stent cage in position for a short period of time to assess the risk of reocclusion of the vessel before deciding whether to detach and deploy the stent cage as an implant. If the physician chooses not to deploy the stent cage, it can be re-sheathed and removed from the patient.

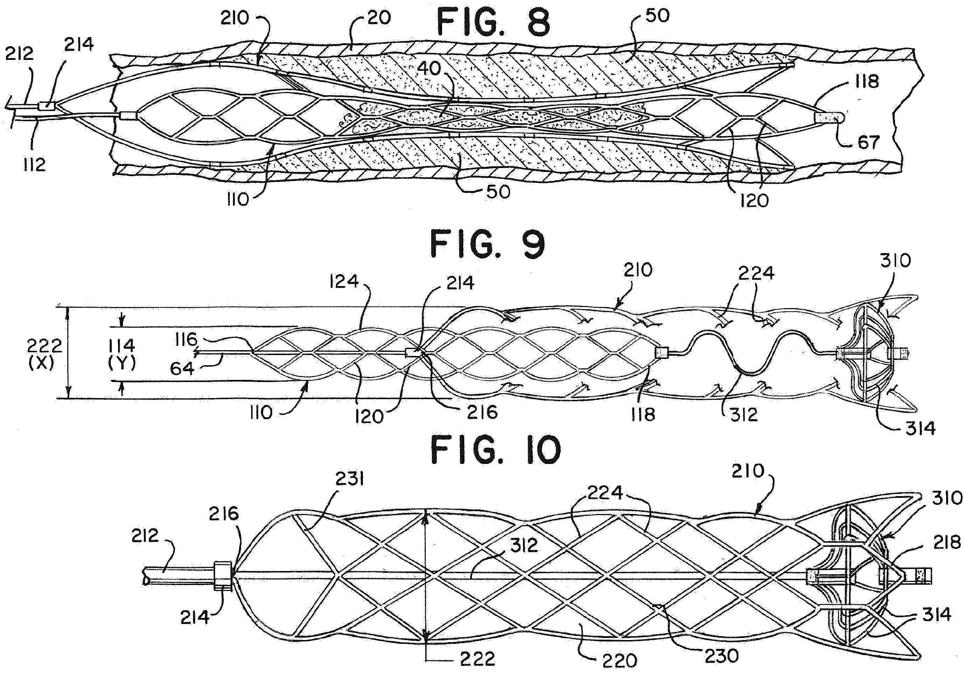

Fig. 8 illustrates the device of Fig. 7 deployed within a portion of a blood vessel 20 having an occlusive thrombus 40 lodged in a stenotic lesion 50. When the maximum radial dimension of the target lesion is less than the maximum radial dimension of the stent cage 210, the cage can be configured to exert an outward radial force when deployed within the lesion. The outer cage can dilate the lesion while the capture section 110 expands to grip the thrombus. A radiopaque marker or coil 67 can mark the extreme distal end of the device so the user can adjust the longitudinal position of the capture section using the second proximal shaft 212 independent of the radial force applied by the outer cage. The stent cage can start to compress and displace the thrombus through the cells formed by the struts 224 as it expands. The large cells can allow the thrombus to escape from compression by displacing a significant portion of the thrombus through the cell openings in the wall of the stent cage. The thrombus could also be drawn through the open distal mouth of the cage with aspiration.

Fig. 9 shows another example of a dual-layer device where the distal end 118 of the elongate body 124 of the capture section 110 overlaps longitudinally with the proximal end 216 of the stent cage 210. As in other examples, the stent cage can have a plurality of interconnected struts 224 forming an outer circumferential network of closed cells. The capture section 110 can be fixed to the shared shaft 64 at its proximal end 116, while the stent cage 210 can be fixed to the shaft 64 distally at the detachment point 214. The proximal capture section 110 can expand to a maximum radial size 114 approximately equal to or less than the maximum radial size 222 of the distal stent cage. In another configuration, the capture section 110 can expand to a maximum radial size 114 slightly larger than the maximum radial size 222 of the stent cage 210 such that the capture section is radially constrained by the stent cage.

The maximum radial dimension of the stent cage when expanded can be at least 20% larger than the maximum radial dimension of the expanded capture section. In other instances, the stent cage can be two or more times the diameter of the capture section. The stent cage can have a network of interconnected struts 224 forming large closed cells for a low radial force along the distal length of the device.

The capture section can be a framework of struts 120 organized in to a series of adjacent segments. The struts can be organized into closed cells or could contain one or more bends or undulations along their length such that they engage different portions of a clot or thrombus. The cells and/or bends can be of differing sizes at different longitudinal sections of the capture section. In one example, segments of the capture segment may consist of struts 120 and cells in a flat pattern which is then configured into a wave or undulating shape 312 when viewed from the side. Alternately, the adjacent segments could be aligned longitudinally to collapse the struts of the segments when subjected to longitudinal tensile or compressive loads. Such variations in the radial force exerted by the capture section can embed or pinch the clot between differing portions of the framework of struts. The interpenetration and pinching could also be induced as the device is later drawn and collapsed back into an intermediate or access catheter after capturing the clot. The pinching action can be useful for increasing the device's grip on fibrin-rich clots. The pinching action may also elongate the clot and pull it away from the walls of the lesion or vessel, thus reducing the required dislodging force.

The device can have a fragment protection element 310 downstream of the capture section 110 and situated in the interior of the stent cage 210. The filter element 314 of the fragment protection element 310 provides a physical barrier to any embolus from the clot retrieval procedure. A flexible connecting member 312 can connect the filter element 314 to the distal end 118 of the capture section, meaning the longitudinal position of the protection element is governed by the proximal capture section shaft 112.

In another example of a clot retrieval and stenting device 200 illustrated in Fig. 10, the expandable outer cage can also be configured to engage with and extract a clot. The device can have an outer cage 210 which has a structure to embed in and capture an occlusive clot when expanded from a delivery configuration to a deployed configuration. The outer cage can have a proximal end 216, a distal end 218, and a shaft 212 for articulation of the device. A detachment point 214 can be located at the intersection of the distal end of the shaft and the proximal end of the outer cage can be configured to disengage the outer cage to implant it as a stent.

The outer cage 210 can have a series of interconnected struts 224 forming an outer circumferential network of large closed cells. The interconnected struts could include connecting members running through the interior of the outer cage to provide further surfaces to capture and grip the clot. The struts could be a shape memory allow such as Nitinol, allowing it to expand to a desired maximum radial size 222 when deployed in a vessel. The interconnected struts cut in such a way as to engage and grip a clot during a standard thrombectomy procedure. During this engagement, the clot could be partially located in the cell openings of the interconnected struts 224 and partially in the reception space or inner lumen 220 of the outer cage.

Similar to previous designs disclosed in this description, the retrieval and stenting device 200 can have a fragment protection element 310 which can be deployed approximate the distal end of the outer cage to provide a physical barrier for protecting against the downstream migration of debris liberated during the clot retrieval procedure. The fragment protection element can be connected to the shaft 212 by a flexible connecting member 312. The connecting member can be an extension of shaft 212, or itself have struts or be of an undulating wave pattern which can help in gripping a clot as it expands and contracts under the forces of clot retrieval.

Once retrieval attempts with the device 200 have achieved a desired level of vessel recanalization, the physician may wish to further treat a region of stenosis, such as an ICAD lesion. The device could be cleaned with saline or other media and reintroduced to a target site using the delivery system of the thrombectomy procedure or other suitable means known in the art. When advanced across the lesion, the outer cage 210 can be expanded and then released by actuating the detachment point 214. The maximum diameter or radial size 222 of the outer cage when deployed can be designed so as to minimize the chronic outward force imparted on the stenosis, which is important for the often-fragile vessels of the neurovascular. In one example, the maximum diameter of the deployed outer cage can be configured to be approximately 2.5 mm but in other examples could be up to 4.0 mm. Once implanted as a stent, the shaft 212 can be drawn to pull the connecting member 312 and retrieve the fragment protection element 310 from the patient.

The outer cage 210 and capture section 110 shown in the figures and discussed herein are used to illustrate single aspects of the present invention. Of course, the present invention can be applied to outer cages and capture sections of a variety of shapes and sizes and could be made from a single section or from multiple sections.

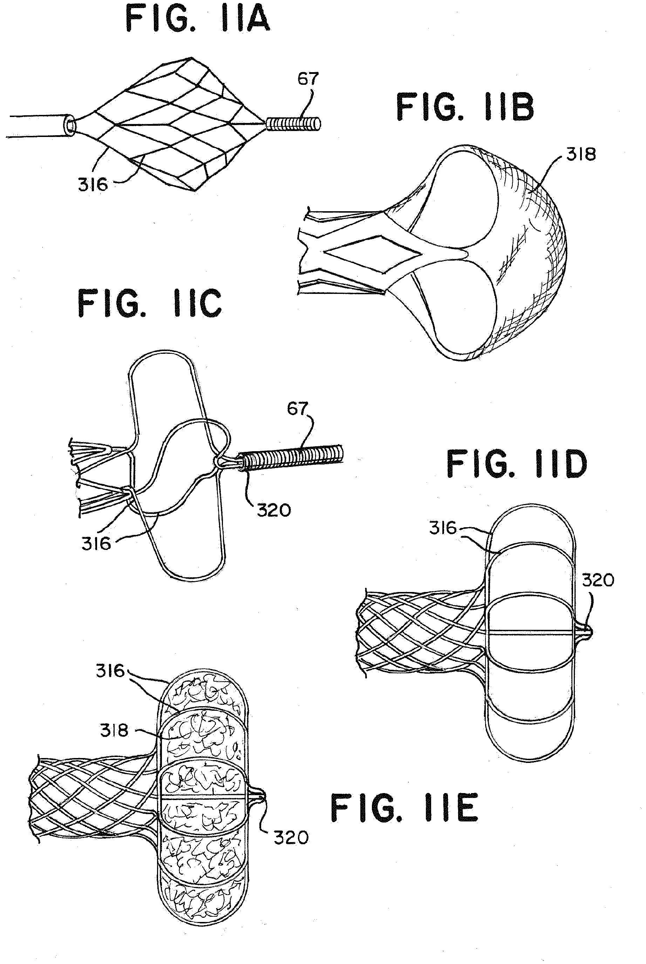

Fig. 11a - Fig. 11e show various alternative designs for the construction of the fragment protection element 310. The fragment protection feature can be created by the attachment of shaped wires or struts 316. The wires or struts can be a number of shapes or orientations to better provide coverage for the open end of the stent cage when the device is deployed. In some examples illustrated in Figs. 11a, 11c, and 11d, the wires or struts are organized in to a three-dimensional structure, where the wires or struts flare radially outward to form a shape equal to or larger than the cross section of the inner expandable capture section of the device. The wires or struts can be an interconnected network, or they could form a spoke-type arrangement extending from a common hub 320 of the fragment protector. In another set of examples illustrated in Figs. 11b and 11e, the wires or struts are combined together to create an intertwined fibrous filter mesh 318. The mesh can be woven in an organized pattern, or the wires or fibers can be entangled into a bundle.

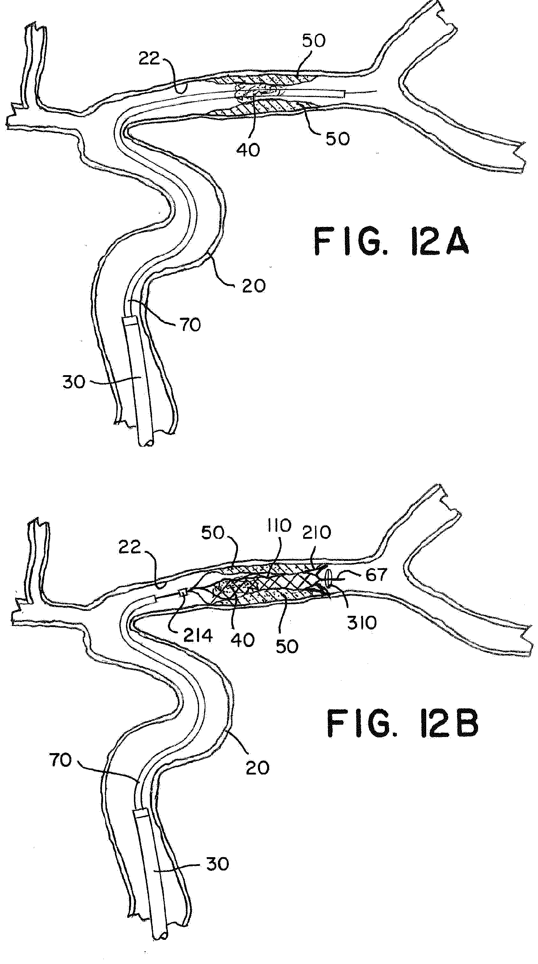

Fig. 12a-d show one possible use sequence for a device as disclosed herein. Fig. 12a shows an example of the device navigated to a target vessel 40 within the neurovascular. The delivery system can use an access catheter 30 to access the site and the device can be loaded in a microcatheter 70 for deployment. The target site can be a vessel occluded as shown, with an obstructive clot 40 lodged in an area where the vessel lumen 22 is also narrowed by a region of intercranial stenosis in the form of a lesion 50 from the buildup of atherosclerotic plaque.

The microcatheter 70 can be advanced until it is positioned distal to the clot 40. Once in the proper position, the microcatheter can be withdrawn proximally, allowing the stent cage 210 and capture portion 110 to expand within and either side of the clot, as shown in Fig. 12b. Expansion of the stent cage displaces the clot and dilates the lumen 22 of the vessel 20 so that flow is restored. The struts of the capture section 110 engage with and grip the clot.

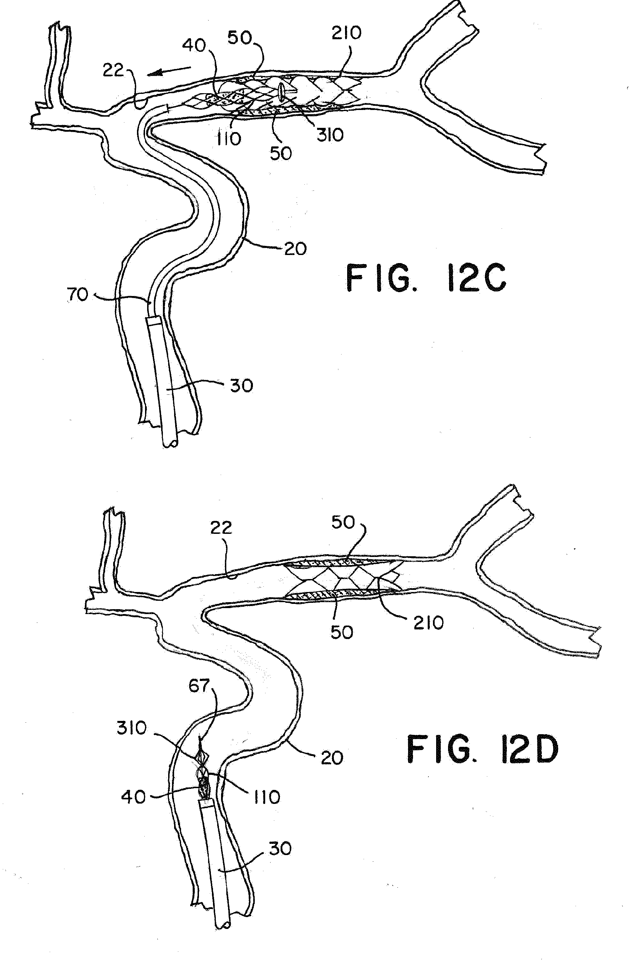

In Fig. 12c the capture section 110 has begun to extract the clot 40 proximally from the target vessel 20. Extraction can be accompanied by aspiration directed from a separate aspiration catheter or from the guide or access catheter 30 to help sustain a firm grip on the clot and avoid fragment loss and migration. The structure of the capture section can have adjacent segments which differ in shape such that the forces exerted on the clot are variable when the capture section is retrieved back in to the intermediate or access catheter. The variance in forces between adjacent sections can result in a pinching action which is more effective at maintaining grip on particularly firm or irregular clot morphologies. In some cases, the stent cage 210 will be withdrawn into the access catheter 30 with the capture section 110 as a unit, or they may be manipulated and positioned separately when multiple shafts are used. As an alternative, the device can be held in position and at least partially re-sheathed by distally advancing the access catheter.

With the region of stenosis identified, the user has the ability to disengage the stent cage 210 as an implant in the lesion 50 as visualized in Fig. 12d. After the occlusive clot has been safely withdrawn, the device can be advanced back across the stenosis if not already in position, such that the stent cage is aligned with the lesion in the vessel. The outer diameter of the stent cage and the shape of the outer cage framework chosen so that an effective amount of support is given to the target vessel 20 so that the reconstituted flow path can be maintained through the narrowed region of stenosis. For example, the desired flow path diameter in the stented region is reached when a constricted first diameter in portion of the vessel containing the lesion is increased to 75% of a second diameter in a portion of the vessel adjacent to the first. At the completion of the procedure the remainder of the dual layer device can be withdrawn from the patient.

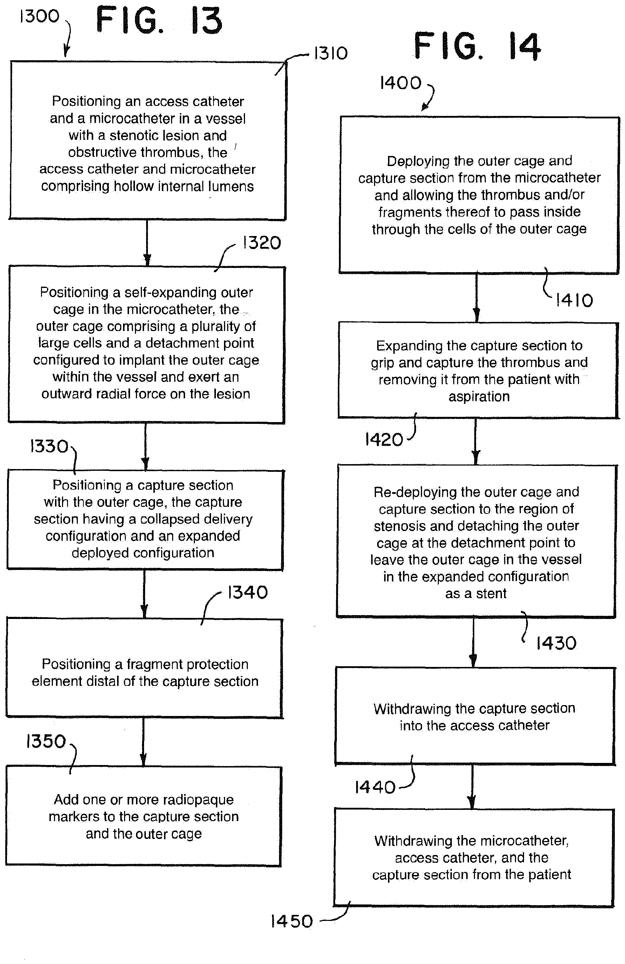

Fig. 13 and Fig. 14 are flow diagrams each comprising method steps for performing a mechanical thrombectomy procedure. The method steps can be implemented by any of the devices and/or apparatus described herein.

Referring to method 1300 outlined in Fig. 13, in step 1310 access to a patient's vascular is gained through traditionally-known techniques and an access catheter and a microcatheter having hollow internal lumens are positioned in a vessel in the neurovascular with a stenotic lesion and occlusive clot. The access catheter can be an outer guide sheath or aspiration catheter as described herein or an alternative known to a person of ordinary skill in the art. A dual layer thrombectomy and stenting device can be positioned in the microcatheter. In step 1320, the device can have a self-expanding outer cage comprising a plurality of large cell openings and a detachment point configured to release the outer cage from the remainder of the device to implant it as a stent. In step 1330, the second layer can comprise an inner capture section within the outer cage, the inner capture section having a maximum radial size less than, equal to, or slightly larger than a maximum radial size of the outer cage depending on the application. The capture section can have an expandable network of struts for gripping the clot and dislodging it from the vessel.

The device can further have additional components to facilitate intravascular procedures. In step 1340 a fragment protection element can be positioned distal to the capture portion to occupy space across the vessel lumen and prevent distal egress of fragments liberated during the procedure. The protection element could be attached to the capture section with a flexible shaft or wire and could take any of a number of forms familiar to this purpose to those of skill in the art. Additionally, a further step 1350 could involve adding one or more radiopaque markers to important points on the device such that they are readily visible under fluoroscopy during the procedure. For example, markers could be added to the proximal and distal ends of the device to mark the terminal treatment points during the operation.

Referring to method 1400 outlined in Fig. 14, in step 1410 the microcatheter is advanced from the access catheter towards and across an occlusive clot and unsheathing the outer cage and capture section to expand from the microcatheter. The large cells of the outer cage displace the thrombus to the internal reception space. In step 1420, the capture section expands to engage and grip the clot. Step 1420 can also include the step of retracting the thrombectomy and stenting device with the captured clot proximally back into the lumen of the microcatheter and/or access catheter for removal from the patient. The preceding steps can also be repeated as necessary if additional capture attempts are needed to sufficiently clear the vessel.

To treat the narrowed region of stenosis in the vessel, the user can re-deploy the outer cage and capture section to the target site and align them with the lesion, as in step 1430. The detachment point can be actuated to leave the outer cage in the vessel in the expanded state as an implanted stent. In steps 1440 and 1450, the remainder of the device, including the capture section, can be withdrawn into the access catheter and removed from the patient.

The invention is not necessarily limited to the examples described, which can be varied in construction and detail. The terms "distal" and "proximal" are used throughout the preceding description and are meant to refer to a positions and directions relative to a treating physician. As such, "distal" or distally" refer to a position distant to or a direction away from the physician. Similarly, "proximal" or "proximally" refer to a position near to or a direction towards the physician. Furthermore, the singular forms "a," "an," and "the" include plural referents unless the context clearly dictates otherwise.

By "comprising" or "containing" or "including" is meant that at least the named compound, element, particle, or method step is present in the composition or article or method, but does not exclude the presence of other compounds, materials, particles, method steps, even if the other such compounds, material, particles, method steps have the same function as what is named.

In describing example embodiments, terminology has been resorted to for the sake of clarity. It is intended that each term contemplates its broadest meaning as understood by those skilled in the art and includes all technical equivalents that operate in a similar manner to accomplish a similar purpose. It is also to be understood that the mention of one or more steps of a method does not preclude the presence of additional method steps or intervening method steps between those steps expressly identified. Some steps of a method can be performed in a different order than those described herein without departing from the scope of the disclosed technology. Similarly, it is also to be understood that the mention of one or more components in a device or system does not preclude the presence of additional components or intervening components between those components expressly identified. For clarity and conciseness, not all possible combinations have been listed.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

The descriptions contained herein are examples of embodiments of the invention and are not intended in any way to limit the scope of the invention. While particular examples of the present invention are described, various modifications to devices and methods can be made without departing from the scope and spirit of the invention. For example, while the examples described herein refer to particular components, the invention includes other examples utilizing various combinations of components to achieve a described functionality, utilizing alternative materials to achieve a described functionality, combining components from the various examples, combining components from the various example with known components, etc. The invention contemplates substitutions of component parts illustrated herein with other well-known and commercially-available products. To those having ordinary skill in the art to which this invention relates, these modifications are often apparent and are intended to be within the scope of the claims which follow.

### Aspects of the invention:

1. A method for treating a stenotic lesion and obstructive thrombus, the method comprising the steps of:
   positioning an access catheter and a microcatheter in a vessel with the stenotic lesion and obstructive thrombus, the access catheter and microcatheter comprising hollow internal lumens;
   positioning a self-expanding outer cage in the microcatheter sized to exert an outward radial force on the lesion, the outer cage comprising a plurality of large cells and a detachment point configured to implant the outer cage within the vessel;
   positioning an expandable capture section with the outer cage, the capture section having a collapsed delivery configuration and an expanded deployed configuration;
   deploying the outer cage and capture section from the microcatheter to allow the thrombus and/or fragments thereof to pass radially inwards through cells of the outer cage;
   deploying the capture section from the microcatheter to capture the thrombus;
   pinching the thrombus between a portion of one or more of the capture section, outer cage, access catheter, and microcatheter;
   detaching the outer cage at the detachment point and leaving the outer cage in the vessel as a stent in the expanded configuration; and withdrawing the capture section with the thrombus into the access catheter; and
   withdrawing the microcatheter, access catheter, and the capture section with captured thrombus from the patient.
2. The method of aspect 1, further comprising the step of providing a distal fragment protection element.

## Claims

1. A device for treating occlusions and stenotic lesions in the vasculature, the device comprising:
a capture section comprising:
an elongate body comprising a plurality of struts;
a proximal end;
a distal end;
a collapsed delivery configuration; and
an expanded deployed configuration wherein the occlusion is pinched by the plurality of struts; and
a stent cage comprising a collapsed delivery configuration and an expanded deployed configuration;
wherein the stent cage is configured to be detachable from the device to remain implanted in the lesion when the capture section is removed from the lesion.

2. The device of claim 1, wherein the stent cage is a stent.

3. The device of claim 1, wherein the capture section is a stentriever configured to engage one or more portions of the occlusive thrombus on movement from the collapsed delivery configuration to the expanded deployed configuration.

4. The device of claim 1, wherein the stent cage and the capture section share a common proximal shaft.

5. The device of claim 1, wherein the stent cage further comprises a plurality of interconnected struts forming an outer circumferential network of closed cells, optionally wherein at least two of the plurality of interconnected struts are longitudinally aligned.

6. The device of claim 1, wherein the capture section comprises a strut framework forming an outer network of closed cells, wherein at least a portion of the strut framework is configured to embed and pinch at least a portion of the thrombus in the expanded deployed configuration as the capture section is drawn back into an outer catheter.

7. The device of claim 1, further comprising a fragment protection element at the distal end of capture section, the fragment protection element comprising:
a connecting member ; and
a distal fragment filter expandable to at least the same radial size as the capture section in the expanded deployed configuration.

8. The device of claim 1, wherein the stent cage is configured to exert an outward radial force when deployed to expand within a lesion.

9. The device of claim 1, wherein a first proximal shaft is capable of articulating the capture section and a second proximal shaft is capable of articulating the stent cage.

10. A device for treating an occlusive thrombus and areas of stenosis in the vasculature, the device comprising:
a proximal shaft; and
an outer cage comprising;
a substantially tubular structure comprising a collapsed delivery configuration, an expanded deployed configuration; and a maximum radial dimension;
wherein the outer cage is configured to exert an outward radial force to embed and pinch the occlusive thrombus when deployed to expand within a region of stenosis;
wherein the outer cage is further configured to be detachable from the device to remain implanted in the stenosis.

11. The device of claim 10, wherein in the expanded deployed state the expanded maximum radial dimension of the outer cage is approximately 6.0 mm in diameter.

12. The device of claim 10, wherein the distal end of the outer cage flares to a radial dimension larger than the maximum radial dimension of the outer cage.

13. The device of claim 10, wherein the outer cage is an implantable stent.

14. The device of claim 10, wherein the outer cage further comprises a structure of interconnected struts, optionally wherein (i) the interconnected struts of the outer cage are configured to engage at least a portion of the thrombus in the expanded deployed configuration, or (ii) the interconnected struts of the outer cage are configured to embed and pinch at least a portion of the thrombus in the expanded deployed configuration.

15. The device of claim 10, further comprising a distal fragment protection element fixedly connected to the proximal shaft by a connecting member.
